# EUROPEAN PATENT SPECIFICATION

(11) **EP 3 929 585 B1**
(45) Date of publication and mention of the grant of the patent: **25.12.2024**
(21) Application number: 21179159.5
(22) Date of filing: 12.06.2021
(51) Int. Cl.: G01N 33/543

(54) **A METHOD AND A BIOSENSING DEVICE FOR PEPTIDES AND PROTEINS DETECTION AND QUANTIFICATION**
EINE METHODE UND EIN BIOSENSING-GERÄT ZUR ERKENNUNG UND QUANTIFIZIERUNG VON PEPTIDEN UND PROTEINEN
PROCÉDÉ ET DISPOSITIF DE BIOSENSEUR POUR LA DÉTECTION ET LA QUANTIFICATION DE PEPTIDES ET DE PROTÉINES

(30) Priority: 28.06.2020 PT 2020116532; 28.06.2020 EP 20182756
(43) Date of publication of application: 29.12.2021
(73) Proprietor: Instituto Superior de Engenharia do Porto, 4249-015 Porto (PT)
(72) Inventor: Moreira, Felismina, 4435-056 Rio Tinto (PT); Sales, Maria, 4470-719 Vila Nova da Telha (PT)
(74) Representative: Patentree

(56) References cited:
- WO-A1-2019/115273
- US-A1- 2009 110 601
- PENG YANFEN ET AL: "Covalent Binding of Antibodies to Cellulose Paper Discs and Their Applications in Naked-eye Colorimetric Immunoassays", JOURNAL OF VISUALIZED EXPERIMENTS, no. 116, 21 October 2016 (2016-10-21), XP055967191, DOI: 10.3791/54111
- WANG XIU ET AL: "Label-free determination of thyroglobulin using template-probe double imprinted composites", SENSORS AND ACTUATORS B: CHEMICAL, ELSEVIER BV, NL, vol. 313, 26 March 2020 (2020-03-26), XP086135736, ISSN: 0925-4005, [retrieved on 20200326], DOI: 10.1016/J.SNB.2020.128028
- PEREIRA MARTA V. ET AL: "Paper-Based Platform with an In Situ Molecularly Imprinted Polymer for [beta]-Amyloid", ACS OMEGA, vol. 5, no. 21, 2 June 2020 (2020-06-02), US, pages 12057 - 12066, XP055852370, ISSN: 2470-1343, Retrieved from the Internet <URL:https://pubs.acs.org/doi/pdf/10.1021/acsomega.0c00062> DOI: 10.1021/acsomega.0c00062
- FERREIRA NÁDIA S ET AL: "New electrochemically-derived plastic antibody on a simple conductive paper support for protein detection: Application to BSA", SENSORS AND ACTUATORS B: CHEMICAL, ELSEVIER BV, NL, vol. 243, 15 December 2016 (2016-12-15), pages 1127 - 1136, XP029915563, ISSN: 0925-4005, DOI: 10.1016/J.SNB.2016.12.074
- GIZEM ERTÜRK ET AL: "Molecular Imprinting Techniques Used for the Preparation of Biosensors", SENSORS, vol. 17, no. 2, 4 February 2017 (2017-02-04), pages 288, XP055592498, DOI: 10.3390/s17020288
- CHEVALIER F ET AL: "Proteomic capacity of recent fluorescent dyes for protein staining", PHYTOCHEMISTRY, ELSEVIER, AMSTERDAM , NL, vol. 65, no. 11, 1 June 2004 (2004-06-01), pages 1499 - 1506, XP004522311, ISSN: 0031-9422, DOI: 10.1016/J.PHYTOCHEM.2004.04.019
- KONG QINGKUN ET AL: "A novel microfluidic paper-based colorimetric sensor based on molecularly imprinted polymer membranes for highly selective and sensitive detection of bisphenol A", SENSORS AND ACTUATORS B: CHEMICAL, ELSEVIER BV, NL, vol. 243, 27 November 2016 (2016-11-27), pages 130 - 136, XP029915436, ISSN: 0925-4005, DOI: 10.1016/J.SNB.2016.11.146
- AKBULUT YELIZ ET AL: "A molecularly imprinted whatman paper for clinical detection of propranolol", SENSORS AND ACTUATORS B: CHEMICAL, ELSEVIER BV, NL, vol. 304, 20 October 2019 (2019-10-20), XP085938591, ISSN: 0925-4005, [retrieved on 20191020], DOI: 10.1016/J.SNB.2019.127276

## Description

### FIELD OF THE INVENTION

The present invention relates to a method and to a biosensing device for detecting and quantifying selected proteins or peptides of interest (POI). This device is based on a paper colour test-strip platform having the respective biosensor built-in by assembling a molecular imprinting polymer (MIP) recognized as a plastic antibody on the paper strip thus, acting as the biorecognition element of the device. The target POI can be identified and quantified by conventional staining methods.

The device of the invention is useful for detecting and quantifying peptides or proteins of interest (POI) present in a given biological sample by visual comparison of colour change providing an independent read-out method, which is equipment-free, accurate, simple, low cost, having a long shelf-life able to detect very low amounts of peptides or proteins in a sample that can be used for tracking any (bio)molecule of interest, such as the used in health, environment and food safety industries.

The present invention is in the area of biosensors, biochemistry applied to protein/peptide detection.

### STATE-OF-THE-ART

Colorimetric or optical biosensors have been extensively studied in sensing biomolecules, metal ions, and other compounds. These biosensors have shown great advantages over conventional assays, particularly concerning their visible response signals, since being possible to observe the results directly by naked eye, there is no need of special equipment for readout.

Several colorimetric assays have been reported in the literature for proteins detection in point-of-care. These includes the well-known lateral flow assays (LFAs). These assays show advantage in terms of portability, free - readout and equipment free. However, this approach is complex, because it uses a porous membrane with specific antibodies or proteins immobilized in lines. Also, LFAs show other concerns, as it is based in a "one-step" assay, in which the biosensor surface is not easily washed and, subsequently, may suffer from interference from sample components that pre-block the strips. Besides that, these assays demonstrate a qualitative and semi-quantitative nature. In addition, sometimes it is needed to label the antibody for increasing the sensitivity becoming a more complicated and expansive assay, especially accounting the needs of a very selective antibody.

A similar tool to LFA using cellulose as a support material is the dipstick-based sensing system. When the dipstick gets in touch with the sample for example of urine or of other physiological fluid, a colour change in the stick is generated.

These methods are a simple but of offer a response of semi-quantitative nature, thereby limiting the accuracy of the analytical data generated.

In addition, the methods reported in the literature use as a biorecognition element enzymes or antibodies, becoming the assay expansive and with a limiting lifetime.

Recently, protein immobilization on oxidized paper enhancement was described being further quantified by colorimetric assay with Ponceau dye. The modified cellulose paper is applied as a sensor for protein quantification in urine, a test able to detect levels of proteinuria and even microalbuminuria. However, this method did not present a specific biorecongiton layer, limiting its selectivity (Imamura et al. 2020).

Another interesting disclosure refers to an aptamer-based biochip for protein detection and quantitation which combines the recent biochip technology and the conventional staining methods. This assay shows very promising features in terms of simplicity. However, being the assay based in an aptamer as a biorecognition element and glass as a support material, it becomes a very expensive assay (Lee and Hah 2012).

Natural receptors have been widely used in different areas. However, the stability of such biomolecules is limited, and they can generally not be used under harsh conditions.

Artificial receptors offer promising alternative to natural recognition elements for biosensors since they provide several advantages in terms of long-term storage stability, resistance to high temperatures, potential re-usability, robustness, and ease of preparation.

There is a growing interest to design and develop simple, reliable, rapid, and inexpensive biosensors for monitoring and detecting proteins as biomarkers for several chronic diseases as cancer and neurodegenerative conditions.

Plastic antibodies rely on molecular imprinted polymer technology (MIP technology) wherein a target molecule is imprinted over the surface, thereby generating artificial binding positions for these molecules.

Document X. Wang et al. (2020) discloses a paper-based platform for colorimetric detection of thyroglobulin which is based on the use of a MIP layer for protein recognition and the color change produced upon bringing into contact the platform with a staining solution (i.a. a solution containing tetramethylbenzidin which acts as colorimetric indicator). The paper-based platform of said document is made of a Whatman filter paper (according to the manufacturer it comprises more than 98% alpha-cellulose). In said document, the paper filter is pretreated with hemin/graphene nanosheets to form a composite before coating the paper with a MIP layer, and the MIP layer is specific for thyroglobulin.

Documents M.V. Pereira et al. (2020) and N.S. Ferreira et al. (2017) discloses a cellulose paper-based platform for electrochemical detection of peptides/proteins based on the use of a MIP as biorecognition layer. In these documents, the MIP is formed by electropolymerization, which implies that the MIP is built on top of an electrode (graphite) layer deposited on top of the cellulose paper support.

Document G. Ertürk & B. Mattiason (2017) discloses a protocol for grafting a layer of MIP on a surface bearing hydroxilic groups via an intermediate amine-functionalized layer further derivatized with glutardialdehyde. The substrate is a magnetic Fe₃0₄ particle and the target analyte is not defined.

Document F. Chevalier et a. (2004) discloses some dye compounds commonly used for protein staining. It is silent about MIPs and paper-based sensing platforms.

Document WO 2019/115273 discloses computerized techniques for processing results data from colorimetric assays carried out with cellulose paper based test stripes with a MIP as recognition layer. Said document is silent about and intermediate amine layer.

Document Q. Kong et al. (2017) discloses a paper-based microfluidic platform for colorimetric detection of bisphenol A comprising a Whatman cellulose paper filter coated with a MIP layer. The MIP layer is formed by bulk polymerization "in situ" of suitable monomers around a template with a crosslinker and an initiator. In analogy to document X. Wang et al. (2020), the MIP is built on top of a layer of magnetic ferrite nanoparticles used to coat the underlying paper substrate.

Document Y. Peng et al. (2016) discloses a paper-based colorimetric biosensing device, wherein said device comprises a substrate made of cellulose filter paper and a biorecognition layer, wherein the cellulose surface is functionalized with an intermediate layer with surface-active amine groups which are further derivatized with a dialdehyde compound, and with a top (biorecognition) layer comprising antibodies. Said document is silent about molecularly imprinted polymers; i.e. the antibodies are not "plastic antibodies".

The present invention provides a paper-strip device that allows to detect and quantify proteins/peptides of interest (POI) in a very sensitive way, simple and low-cost by coupling the biorecognition function of a plastic antibody imprinted by MIP on the paper strip with colour changes properties thus enabling to detect and quantify said POI without the need of specific equipment, enzymes or antibodies as the biosensors of the prior art above-mentioned, even in the point-of care.

### DESCRIPTION OF THE FIGURES

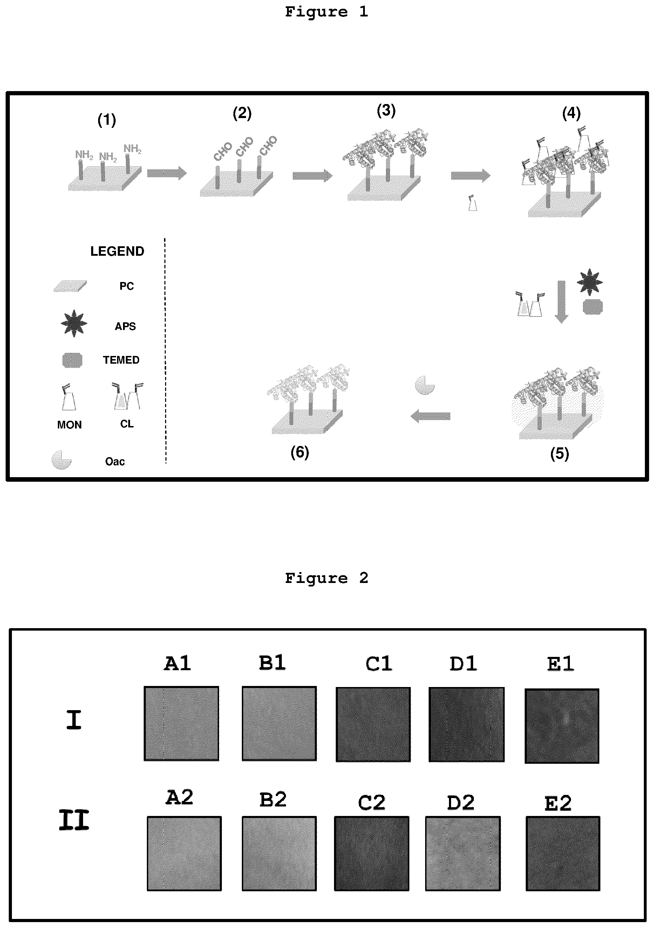

Figure 1 is a scheme representing molecular imprinting of a polymer (MIP) on cellulose paper, where:
   a Represents the formation of an amine layer (1) on cellulose paper (CP),
   b Represents the linkage of an aldehyde group (2) on the amine layer (1), thus forming an amine-aldehyde layer,
   c Represents the binding of a linking peptide POI (3) to the layer formed in (b),
   d Represents the imprinting of the POI (3) as template molecule, by self-organizing the monomeric structures (MON) around a formed POI template (4),
   e Represents the polymerization of monomers (MON) with a cross-linker (CL) with addition of initiators (5),
   f Represents the imprinted sites (6) obtained after the removal of peptide (3) by treatment with oxalic acid (Oac), thus forming a solid matrix template (4).
Figure 2 represents the control of the surface modification of the biosensor formed by the modified CP with the imprinted sites (6) by applying with CB blue staining solution, where:
   I. Before CB staining,
   II. After CB staining,
      A represents CP, wherein A1 is grey and A2 is light blue,
      B represents CP modified with APTES, where B1 is grey and B2 light blue,
      C represents the addition of GLU into APTES/CP wherein C1 is brick red and C2 is brown.

After addition of GLU into APTES/CP, the colour of the solution turned into brick-red instantly. When APTES was mixed with GLU, the amino groups on APTES reacted with GLU to form Schiff bases and led to the formation of APTES dimer, which displays brick-red colour.
D represents the CP test-strip modified with the MIP based biosensor wherein C1 is brown and C2 is greenish-brown,
E represents the CP test-strip modified with the NIP (blank polymer) where in C1 is brown and C2 is greenish brown.

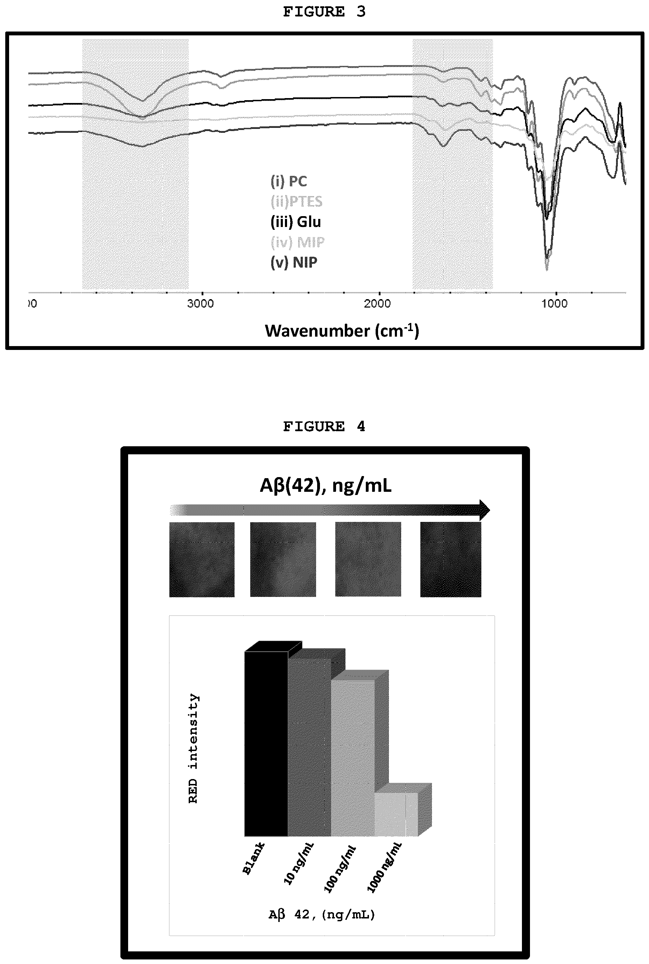

Figure 3 presents the FTIR spectra of different immobilization steps of the CP based sensor, wherein:
i) refers to CP material,
ii) refers to APTES/CP,
iii) refers to GLU/APTES/CP,
iv) refers to MIP/Glu/APTES/CP,
v) refers to NIP/Glu/APTES/CP.

From figure 3 it is possible to observe that absorbances at 3343.1, 2898, 1641, 1428, 1366, 1160.2, 1056, and 897.6 cm⁻¹ are associated with to CP. The peak at 3434.3 cm⁻¹ corresponds to intramolecular OH stretching, including hydrogen bonds and at 2898 cm⁻¹ due to CH and CH2 stretching. Other vibrational peaks at 1642 cm⁻¹ correspond to OH from absorbed water, 1428 cm⁻¹ due to CH2 symmetric bending, 1366 cm⁻¹ due to CH bending, 1160.2 cm⁻¹ due to C-O-C asymmetric stretching 1056 cm⁻¹ due to C-O/C-C stretching and 8976 cm⁻¹ due to the asymmetric out-of-plane stretching vibrations.

Further, it is possible to observe that, after GLU immobilization onto CP modified with APTES an additional band appears at 1566 cm⁻¹ and is representative of C=N stretching. One of the aldehyde group of GLU (-CHO) reacted with the amine previously immobilized on the cellulose paper by means of a nucleophilic addition mechanism forming an imine (-C=N).

After polymerization, a broad band is visible at 1645 cm⁻¹ in MIP/GLU/APTES/CP, being this peak representative of simple mono-substituted C=C stretch.

An additional small band is present at 1548.7 cm⁻¹ representative of amide group from the polymeric matrix. Figure 4 presents the gradient of colour after test-strips incubation with different concentrations of Aβ-42 POI with 0, 10,100 and 1000 ng/mL respectively.

### DESCRIPTION

The present invention, as defined in the claims, relates to a method and to a biosensing device for detecting and quantifying selected biomolecules such as peptides or proteins of interest (POI) present in a biological sample.

The biosensing device is based on a cellulose paper colour test-strip platform built-in by assembling a molecular imprinting polymer treated with said POI on the paper strip, acting as the biorecognition element of the device.

Because of this, it is possible to detect and quantify the POI by direct contact of the biosensor with the sample to be analysed. The target peptide/protein (POI) can be visually detected by conventional protein staining methods and quantified by colour graduation after promoting the contact of the biosensor device with a biological sample having said POI therein.

### 1. The biosensing device

The biosensing device, as defined on the claims, is based on a cellulose paper (CP) test-strip support comprising a plastic antibody acting as the biorecognition element of the device, which is specific for a given POI.

The biosensing device further comprises adequate staining compounds able to induce chemical changes upon the modified cellulose substrate (CP) with the biorecognition element, attributing it the ability to react with a given peptide or protein present in a biological sample and to produce a colour change therein, when in presence of a target peptide (POI).

These staining compounds can be provided apart of the biosensor device, for example in the format of a kit, thus comprising the cellulose paper (CP) test-strip support and the specific plastic antibody as a first element of the kit, and the staining solution as a second element of the kit.

The kit for detecting and quantifying a selected peptide or protein of interest (POI), as defined in the claims, is provided with a third element, which is a washing solution for removing the excess of staining solution.

Therefore, by using the above-mentioned kit, the method of the invention provides an easy, reliable, and accurate way to detect and quantify a given POI present in a biological sample in the point-of care.

In the scope of the present invention a "plastic antibody" means a biorecognition layer built by molecular imprinting polymer (MIP) technology.

MIP is the 3-D or 2-D imprint of a certain molecule in a rigid polymeric matrix build with synthetic materials of organic (vinyl functional derivatives) or inorganic (silica derivatives) nature.

Biomimetic materials are mostly prepared by MIP technology, where the target molecule is imprinted over the surface, thereby generating artificial binding positions for these molecules.

In the scope of the present invention, the imprinted molecule is a peptide of interest (POI), and therefore, the biosensing device of the invention is able to recognize said POI after having contacted a biological sample comprising it even in very low concentrations.

The recognition of a given POI produces a change of colour and the intensity of the colour generated gives an accurate quantification of said POI in the analysed biological sample, when compared by a standard with a colour gradient.

### 1.1 The cellulose paper test-strip

The cellulose material that can be used as support material of the biosensing device is of the type qualitative filter paper, circles, diam. 25 mm, such as the one provided by Whatman.

Other cellulose paper (CP) that can be used in the invention are Whatman^{®} cellulose chromatography papers, Whatman^{®} qualitative filter paper, Grade 470, nitrocellulose film slides, Whatman Grade 4(qualitative filter paper circles), GE healthcare Whatman^{™} cellulose filter paper, and cellulose and polyester fibre (cleanroom paper wipers).

The cellulose paper can be cut into strips of approx. 1×1 cm² slides.

### 1.2 The amine-layer

Strips of cellulose paper (CP) are treated with a compound (1) capable of providing an amine group (-NH₂ ) for the formation of the amine layer onto the test-strips as described above.

As defined in the claims, compounds capable of providing an amine group for the formation of the amine layer can be selected from the group preferably comprising 3-aminopropyltriethoxysilane (APTES), ethylenediamine, phenylenediamine, poly(diallyl dimethyl ammonium chloride, poly(vinylamine) hydrochloride, poly(L-lysine hydrobromide), polyethyleneimine hydrochloride, poly(allylamine hydrochloride), poly(4-aminostyrene), poly(allylamine), poly (ethylene glycol) bis (2-aminoethyl), chitosan, poly(I-lysine hydrobromide).

### 1.3 The aldehyde-layer

The CP strips with an amine-layer (1) prepared as above is further incubated with a solution comprising a dialdehyde compound (2) capable of providing two terminal aldehyde functional groups (-CHO) to form an aldehyde-layer.

The two terminal aldehyde groups of compound (2) react with the amine group of the amine-layer (1) by means of a nucleophilic addition mechanism forming an imine (-C=N) group thus resulting in an aldehyde-layer (2).

Dialdehyde compounds adequate for the invention include glutaraldehyde (GLU), benzene-1,4-dialdehyde, and succindialdehyde.

### 1.4 Binding of a peptide (POI)

The biorecognition element of the device is produced by molecularly imprinting of a POI (3) onto a treated CP-strip having an amine-layer modified with an aldehyde-layer.

In the scope of the present invention, any protein or peptide of interest (POI) suitable for the invention include but are not limited to peptide Aβ-42, cardiac myoglobin, troponin T, CK-MB isoenzyme, cancer MUC-1 protein, carcinoembryonic antigen and carbohydrate antigen (CA 19-9), neurodegenerative biomarkers such as amyloid β42, Tau protein, interleukin, antibodies such as SARS-CoV-2 (2019-nCoV), antibodies for malaria, zika and dengue virus and respective antigens, viral proteins SARS-CoV-2 (2019-nCoV) antigen), bacterial proteins such as protein A, serum human albumin amongst others.

Compound (2) comprises two free aldehydes groups, one reacting with the amine group of CP-strip as described above, and the other reacting with the amine groups of a POI (3) thus, also forming an imine group. Consequently, the protein/peptide is attached to the previously treated CP-strip as described above.

### 1.5 The biorecognition layer

The biorecognition layer is made by assembling a molecular imprinting polymer (MIP) with a selected POI (3) on the paper strip. The imprinted sites (6) are obtained once the peptide/protein (3) is removed.

The biosensing device based on a colorimetric test-strip for detecting and quantifying selected proteins or peptides (POI) thus comprises a modified support of cellulose filter paper (CP) having a layer formed by amine groups (1) linked to aldehyde groups (2), a and biorecognition layer built by molecular imprinting polymer technology comprising imprinted sites (6) for a given POI (3) able to capture said POI (3) when contacting a biological sample comprising it.

The biosensing device of the invention is able detect and quantify proteins/peptides of interest (POI) present in a biological sample by visual comparison of colour change providing an independent read-out method, which is equipment-free, accurate, simple, low cost, having a long shelf-life able to detect very low amounts of peptides or proteins after staining with conventional staining compositions and methods.

### 1.6 Staining compositions

Detection and quantitation of the peptide (POI) bound to the biosensor can be assessed by staining the test-strip with conventional protein dyes and staining methods.

Suitable dyes for the invention include but are not limited to Coomassie brilliant blue R-250, bromophenol, methylene blue, silver nitrate and zinc nitrate, fluorescent dyes as eosin, rhodamine, and Nile red and brilliant cresyl blue oxazone.

### 1.7 Washing compositions

After staining the biosensing device of the invention for the detection and quantification of a POI the excess of dye can be removed by washing with an inert solution such as acetate, citrate, phosphate, ethanol, methanol and water.

The biosensing device can be provided as a kit comprising the test-strip having imprinted sites for a given POI, a container with a staining solution and another container with a washing solution. Preferably, the kit comprises several test-strips and each container comprise an amount of the respective solution for staining and washing the correspondent number of test-strips included in the package of the kit. Each test-strip is enough for performing the method of detecting and quantifying the specific POI for which the plastic antibody of the biosensor is designed.

### 2. A process for preparing a biosensing device

The biosensing device of the invention, as described in the previous section, is prepared according to the following steps, and as shown in Fig.1 and defined in the claims:
A Providing a cellulose paper (CP) strip support,
b Forming an amine layer (1) on cellulose paper (CP), by treating the CP-strip support with an amine compound selected from the group 3-aminopropyltriethoxysilane (APTES), ethylenediamine, phenylenediamine, poly(diallyldimethyl ammonium chloride, poly(vinylamine) hydrochloride, poly(L-lysine hydrobromide), polyethyleneimine hydrochloride, poly(allylamine hydrochloride), poly(4-aminostyrene), poly(allylamine), polyethylene glycol)bis(2-aminoethyl), chitosan, poly(I-lysine hydrobromide),
c Incubating the amine layer (1) with a dialdehyde compound able to provide two terminal aldehyde functional groups (-CHO) forming an amine-aldehyde layer (2),
d Binding of a POI (3) to the amine-aldehyde layer formed in (c),
e Molecularly imprinting of the POI (3) by polymerization of a monomeric structure (MON) around a template (4) with a cross-linker (CL) and an initiator (5),
f Forming the imprinted sites (6) by removal of POI (3).

### 2.1 Preparation of cellulose material

Cellulose material as described in the previous section is cut into strips of approx. 1×1 cm² slides.

Cellulose paper-strips can be advantageously pre-treated by incubation in ethanol absolute during 24 h to remove some organic compounds on its surface.

### 2.2 Formation of an amine-layer (-NH₂ )

The amine-layer (1) is formed by incubating the CP-strips in a solution 10% of a compound (1), able to provide an amine (-NH₂ ) functional group, preferably 3-aminopropyltriethoxysilane (APTES), in 10% of an OH-solution, preferably an ethanolic solution. The monolayer (1) thus obtained has a standing-up composition with the amine groups (-NH₂ ) exposed to the external surface of the PC.

### 2.3 Formation of an aldehyde-layer(-CHO)

The CP-strips having an amine layer (1) are further incubated with a solution comprising a dialdehyde compound (2) able to provide two terminal aldehyde functional groups (-CHO), preferably glutaraldehyde (GLU), to form an aldehyde + amine-layer (1+2) . This step can be performed at room temperature (20-20°C), for 50 minutes at a pH value of approx. 5. After incubation, the thus obtained CP-strips are repeatedly washed with distilled water for removal of undesired excess of residues.

### 2.4 Peptide (POI) immobilization

A peptide of interest (3) is immobilized onto the cellulose paper-strips (PC) treated as explained above by incubation of a solution of said POI (3) in an adequate buffer, such as PBS buffer at a pH value of approx. 7, for around 3h at a low temperature of 3 to 5°C, preferably of 3.5 to 4.5°C, more preferably at 4°C.

The CP-strips with immobilized POI (3) as above described can be washed to remove non-bound POI with an adequate solution such as deionised water and PBS buffer.

### 2.5 Forming the plastic antibody

The plastic antibody of the biosensor is produced by imprinting a selected POI according to any MIP known method, such as the one described by Moreira, F.T.C., et al. (BIOSENSORS & BIOELECTRONICS (28, 243-250, 2011 ).

In the scope of the present invention, molecular imprinting starts by self-organizing the monomeric structures of a polymer monomers (MON) around a template (4) followed by polymerizing the MON with a cross-linker (CL). The polymerization starts with addition of a radicalar initiator (5). The imprinted sites (6) are obtained once the peptide/protein (3) is removed.

Monomers (MON) adequate for the invention include, but are not limited to, acrylamide monomer (AAM), vinyl benzene boric acid, 4-vinyl benzaldehyde, 4-vinyl aniline, tert-butyl p-vinylphenylcarbonate, acrylic acid, methacrylic acid, trifluoromethyl acrylic acid methyl methacrylate, p-vinylbenzoic acid, itaconic acid, 4-ethylstyrene, styrene, 4-vinylpyridine,vinylpyridine, 1-vinylimidazole, acrylamide, 2-acrylamido-2-methyl-1-propane sulfonic acid, 2-hydroxyethyl methacrylate, trans-3-(3-pyridyl)-acrylic acid.

Suitable crosslinkers (CL) for the invention include but not limited to N,N'-methylenebis(acrylamide) (N,N-BISAA), bis-(1-(tert-butylperoxy)-1-methylethyl)-benzene, ethylene glycoldimethacrylate, N,N;-methylenediacrylamide, divinylbenzene, 1,3-diisopropenyl benzene, N,N' -1,4-phenylenediacrylamine, 2,6-bisacryloylamidopyridine, 3,5-bis(acryloylamido)benzoicacid, tetramethylene dimethacrylate, trimethylpropane trimethacrylate.

CP-strips treated as described previously can be imprinted with a given POI by incubating a monomer (MON) with a cross-linker (5), for approx. 1 h at room temperature at a pH of approx. 7.4.

After incubation, a solution of an initiator is added to start the polymerization. The polymerization is carried out at room temperature (20-25°C), for approx. 4 h, after which the sensor is thoroughly washed with deionised water.

Suitable initiators (5) for the invention include but are not limited to ammonium persulphate (APS), tetramethylethylenediamine (TEMED), azobisisobutyronitrile azobisdimethylvaleronitrile, 4,40-azo(4-cyanovaleric acid), benzoylperoxide , dimethylacetal of benzyl.

Finally, the previously attached POI (3) is removed to empty the imprinted sites (6), according to described by Bonini F. et al. (Surface imprinted beads for the recognition of human serum albumin. Biosensors & Bioelectronics, 2007. 22 (9-10) : p. 2322-2328 ) to empty the imprinted sites. This step takes approx. 5h. The imprinted material is then washed and conditioned in PBS buffer, pH 7.4, in order to increase the pH value and to remove the amino acid fractions produced by the treatment.

Suitable chemicals for the template removal from the polymeric matrix include, but are not limited to, oxalic acid, acetic acid, sulphuric acid, methanol and enzymes (trypsin and proteinase K).

### 2.6 Preparation of staining solutions

The staining solutions of the invention can be prepared by any of known methods for this purpose. It can be prepared by dissolving a suitable dye for peptide-staining in a buffer to achieve a staining solution such as bromophenol (blue), methylene blue (blue), silver nitrate (grey) and zinc nitrate (grey), fluorescent dyes as eosin, rhodamine, and Nile blue oxazone.

### 2.7 Preparation of washing solutions

Washing solutions comprise solvents or buffer solutions to remove the excess of staining solution of the test-strips. Typically, they are prepared by mixing prepared in acidic medium as acetate, citrate, phosphate or organic solvents as ethanol, methanol or in water.

### 3. Method of detecting and quantifying a POI in a sample

Detection and quantification of a given peptide (POI) present in a biological sample can be performed by contacting the biosensor of the invention with a biological sample.

Biological samples, in the scope of the present invention are for example, urine, serum and saliva.

Detection and quantitation of the peptide (POI) bound to the biosensor can be assessed by staining the test-strip of the invention with conventional protein dyes.

The results can be assessed either by visual comparison of the colour or by the different colour intensity in the standard curve made with IMAGEJ software with RGB coordinates.

The method of detecting and quantifying a given POI present in a biological sample comprises the following steps:
a) Promoting the contact of a test-strip comprising a plastic antibody for a given POI with a biological sample to be analysed, for at least 30 minutes, preferably for 30 to 60 minutes,
b) Staining the test-strip of (a) with a peptide staining composition, and
c) Comparing the colour generated in the test-strip of (b) with a standard colour gradient for the corresponding peptide staining composition.

Suitable peptide staining compositions for the invention include but are not limited to Coomassie brilliant blue R-250 (CB), bromophenol, methylene blue, silver nitrate and zinc nitrate, fluorescent dyes as eosin, rhodamine, and Nile blue oxazone, as indicated before.

The test-strips are incubated with a peptide staining composition for approx. 30 minutes to promote the development of the colour of the correspondent staining composition. Preferably, at least half of a test-strip is dipped into the biological sample.

Biological samples as plasma or serum can be pre-treated. Serum is the liquid fraction of whole blood and thus it can be collected after blood clotting. The clot is removed by centrifugation and the resulting supernatant, designated serum, is carefully removed using a Pasteur pipette. Plasma is produced when whole blood is collected in tubes treated with an anticoagulant (e.g., EDTA-treated or citratetreated). The blood does not clot in the plasma tube. The cells are removed by centrifugation. The supernatant, designated plasma, is carefully removed from the cell pellet using a Pasteur pipette.

Liquid samples such as urine, serum, plasma, saliva, sweat, can be put directly in contact with the test-strips of the invention.

Different colours are generated by different staining compositions. As an example, the following dyes are presented followed by the corresponding colour generated: Bromophenol (blue), methylene blue (blue), silver nitrate (grey) and zinc nitrate (grey); fluorescent dyes such as eosin, rhodamine, and Nile blue oxazone can also be used.

### EXAMPLES

### Example 1. Preparation of cellulose paper-strips

Cellulose paper-strips (CP) were subjected to a multi-step treatment to obtain the cellulose test-strip based biosensor with a MIP as a biorecognition element.

Cellulose paper Whatman^{®} qualitative filter paper was commercially acquired, and several strips 1×1 cm² slides were obtained by cutting the cellulose paper with a scissor.

Cellulose paper-strips were incubated by immersion in different solutions in a container (2 mL per paper slide) under constant stirring (lateral motion) at room-temperature (20-25°C), in a chamber under protective light conditions, ensuring that the paper-slides were completely submerged in the solution.

### Example 2. Cellulose paper chemical modification

Twenty cellulose strips were used to prepare a biosensor device based on a paper colour test-strip platform.

Twenty cellulose strips were pre-treated by incubation in 25 mL ethanol solution 95% of during 24 h, to remove residual organic compounds on its surface. Incubation was conducted under constant stirring (lateral motion), usually at room-temperature (20-25°C), unless specified otherwise, in a chamber protected from light and ensuring that the paper was completely submerged in the solution.

### 2.1 Cellulose paper modification

### 2.1.1 Linking an amine group

An amine layer was obtained on the pre-treated paper strips by immersing the strips in a 10% solution of 3-aminopropyltriethoxysilane (APTES) in ethanol 10%, during 24 h, with continuous stirring (20 slides were placed into 50 mL flask containing 25 mL of the ethanolic solution).

### 2.1.2 Linking an aldehyde group

The previously aminated test strips with APTES were modified by reaction with glutaraldehyde (GLU). The reaction took place with a 1% solution of GLU, at room temperature, for 50 minutes (20 strips were placed into 50 mL flask containing 25 mL of the MES buffer pH 5.0). The test-strips were then repeatedly washed with distilled water (each strip has been in contact with distilled water for 5 seconds).

### 2.1.3 Linking a protein/peptide

The paper-strips previously modified with GLU were further incubated with 50 µL of 2.5 µg/mL of a peptide Aβ-42 (POI) solution (pH 7.4, in phosphate buffered solution PBS buffer), for 3 h at 4°C in order to form a covalent imine group, for 3h at 4°C. After peptide Aβ-42 immobilization, cellulose strips were washed with deionised water and PBS buffer by immersing in a 1 mL of each solution during ~3 seconds.

### Example 3. Molecular imprinting of the peptide Aβ-42

Paper-strips comprising the peptide Aβ-42 therein attach were washed with deionised water and PBS buffer followed by incubation with 2 mL of 1 mmol L⁻¹ , acrylamide monomer (AAM) and 0.5 mmol L¹ of crosslinker N,N'-methylene-bis(acrylamide) (N,N-BISAA), in PBS solution (pH 7.4).

Next, 20 µlL of 1 mmol L¹ of ammonium persulfate, (APS) and (1% of tetramethylethylenediamine (TEMED) solution prepared in PBS buffer pH 7.4 was added to the previous solution in order to start the polymerization.

The polymerization was carried out at room temperature for 4h, after which each test-strip was thoroughly washed with deionised water and PBS buffer by immersing each slide in a 1 mL of each solution during ~3 seconds.

Finally, the attached peptide was removed by reaction with 0.1 mol L⁻¹, oxalic acid (Oac) to form the imprinted sites, being each strip immersed in 2 mL of Oac solution. This step was carried out at room temperature for 5h. The imprinted material was washed and conditioned in PBS buffer, pH 7.4, to increase the pH and remove the amino acids fractions produced by Oac treatment (see figure 1).

The blank material (NIP) was synthetized in parallel excluding from the procedure the peptide attach step, for comparison purposes.

### Example 4. POI Protein/peptide binding

The binding assay of Aβ-42 to the test-strip based biosensor was verified against incubation with 40 µL of different concentrations of Aβ-42 prepared in PBS buffer pH 7.4, during 30 minutes at room temperature. For this purpose, different test -strips were used:
The test-strip 1 was incubated with PBS buffer,
The test-strip 2 was incubated with 10 ng/mL of Aβ-42,
The test-strip 3 was incubated with 100 ng/mL Aβ-42,
The test-strip 4 was incubated with 1000 ng/mL Aβ-42.

Thereafter, test-strips were washed with PBS buffer (2 mL PBS solution/ slide) and let dry at room temperature for 30 minutes.

### Example 5. Colorimetric detection of the peptide/protein

Detection and quantitation of the POI bound to the biosensor was confirmed by conventional Coomassie brilliant blue R-250 (CB) staining method.

For this purpose, the modified paper strips were incubated with CB staining solution for 30 minutes.

Each strip was incubated with CB solution 0.01% (W/V) prepared in (ethanol- phosphoric acid -water) (EtOH - H₃PO₄ - H₂O; 5:10:85) solution during 30 minutes in order to promote the development of the Blue/green colour.

Then, strips were washed with (EtOH- H₃PO₄ - H₂O; 5:10:85) solution, for 15 minutes, and with PBS buffer at pH 7.4 in order remove the unreacted CB staining solution.

### Example 6. Colour analysis

After taking pictures, the obtained signals were evaluated with Image J program of Windows by measuring the colour coordinates of each image acquired by the RGB colour system (red, green and blue).

| Conditioning solution | Concentration/solution | Supplier |
|---|---|---|
| | | |

| Chemical modification of cellulose slides | | |
|---|---|---|
| Ethanol | 10% (V/V) | Honeywell |
| 3 - aminopropyltriethoxysi lane, APTES | 10% (in ethanolic solution,10%) | Sigma |
| Glutaraldehyde, Glu | 1 % (in MES buffer pH 5) | Fluka |
| MES monohydrate | 0.1 mol L⁻¹ | AppliChem |
| | | |

| Molecular imprinting of the peptide Aβ-42 | | |
|---|---|---|
| Amyloid β-42, Aβ42 | 2.5 µg/mL (in PBS buffer pH 7.4) | GeneScript |
| Acrylamide, AAM | 1 mmol L⁻¹ (in PBS buffer pH 7.4) | Sigma |
| N,N'-methylenebisacrylamide , N,N-BISAA | 0.5 mmol L⁻¹ (in PBS buffer pH 7.4) | Sigma |
| Tetramethylethylenedia mine, TEMED | 1% (in PBS buffer pH 7.4) | Fluka |
| Ammonium persulphate, APS | 1 mmol L¹, (in PBS buffer pH 7.4) | Fluka |
| Oxalic acid, Oac | 0.1 mol L¹ (in PBS buffer pH 7.4) | Fluka |
| PBS buffer (tablets) | 0.01 mol L⁻¹ | Amresco |
| | | |

| Method for detecting peptides | | |
|---|---|---|
| conventional coomassie brilliant blue R-250 (CB) | 0.01% (W/V) in (ethanol-phosphoric acid -water) (EtOH - H3 PO4 - H2 O; 5:10:85) | PANREAC |

### References

Bushman, E.T., Jauk, V.C., Szychowski, J.M., Mazzoni, S., Tita, A.T., Harper, L.M., 2020. Utility of routine dipstick urinalysis for glucose screening as a predictor for gestational diabetes. American Journal of Obstetrics and Gynecology 222(1), S161-S162 .
Carrell, C., Kava, A., Nguyen, M., Menger, R., Munshi, Z., Call, Z., Nussbaum, M., Henry, C., 2019. Beyond the lateral flow assay: A review of paper-based microfluidics. Microelectronic Engineering 206, 45-54 .
Dyerberg, J., Pedersen, L., Aagaard, O., 1976. EVALUATION OF A DIPSTICK TEST FOR GLUCOSE IN URINE. Clinical Chemistry 22(2), 205-210 .
Imamura, A.H., Segato, T.P., de Oliveira, L.J.M., Hassan, A., Crespilho, F.N., Carrilho, E., 2020. Monitoring cellulose oxidation for protein immobilization in paper-based low-cost biosensors. Microchimica Acta 187(5 ).
Lee, G.H., Hah, S.S., 2012. Coomassie blue is sufficient for specific protein detection of aptamer-conjugated chips. Bioorganic & Medicinal Chemistry Letters 22(4), 1520-1522 . Piriya, V.S.A., Joseph, P., Daniel, S., Lakshmanan, S., Kinoshita, T., Muthusamy, S., 2017. Colorimetric sensors for rapid detection of various analytes. Materials Science & Engineering C-Materials for Biological Applications 78, 1231-1245 .
Posthuma-Trumpie, G.A., Korf, J., van Amerongen, A., 2009. Lateral flow (immuno) assay: its strengths, weaknesses, opportunities and threats. A literature survey. Analytical and Bioanalytical Chemistry 393(2), 569-582 .
Soper, S.A., Brown, K., Ellington, A., Frazier, B., Garcia-Manero, G., Gau, V., Gutman, S.I., Hayes, D.F., Korte, B., Landers, J.L., Larson, D., Ligler, F., Majumdar, A., Mascini, M., Nolte, D., Rosenzweig, Z., Wang, J., Wilson, D., 2006. Point-of-care biosensor systems for cancer diagnostics/prognostics. Biosensors & Bioelectronics 21(10), 1932-1942 .

## Claims

1. A biosensing device for detecting and quantifying a selected peptide or protein of interest (POI) (3) present in a biological sample **characterized by** comprising a cellulose paper (CP) test-strip platform comprising an amine-layer (1), and a biorecognition layer for said POI (3) which is built by molecular imprinting polymer (MIP) technology and is capable of generating a change of colour after being stained with an adequate peptide staining solution;
wherein said cellulose paper test-strip platform is a qualitative filter type paper; wherein said amine layer is obtainable by treating the cellulose paper (CP) test-strip platform with a compound selected from a list consisting of: 3-aminopropyltriethoxysilane (APTES), ethylenediamine, phenylenediamine, poly(diallyldimethyl ammonium chloride, poly(vinylamine) hydrochloride, poly(L-lysine hydrobromide), polyethyleneimine hydrochloride, poly(allylamine hydrochloride), poly(4-aminostyrene), poly(allylamine), polyethylene glycol)bis(2-aminoethyl), chitosan, poly(I-lysine hydrobromide);
wherein said amine-layer is further incubated with a solution comprising a dialdehyde compound capable of providing two terminal aldehyde functional groups, thus forming an amine-aldehyde layer (2),
wherein a molecularly imprinted polymer (MIP) layer able to recognize said POI (3) is built on top of said amine-aldehyde layer; and
wherein said POI (3) is selected from a list consisting of: peptide Aβ-42, cardiac myoglobin, troponin T, CK-MB isoenzyme, cancer MUC-1 protein, carcinoembryonic antigen and carbohydrate antigen (CA 19-9), neurodegenerative biomarkers such as amyloid β42, Tau protein, interleukin, antibodies such as SARS-CoV-2 (2019-nCoV), antibodies for malaria, zika and dengue virus and respective antigens, viral proteins SARS-CoV-2 (2019-nCoV) antigen), bacterial proteins such as protein A, and serum human albumin.

2. The biosensing device according to the previous claim **characterized by** the cellulose paper (CP) material is selected from a group comprising Whatman^{®} cellulose chromatography papers, Whatman^{®} qualitative filter paper, Grade 470, nitrocellulose film slides, Whatman Grade 4 qualitative filter paper circles, GE healthcare Whatman^{™} cellulose filter paper, and cellulose and polyester fibre cleanroom paper wipers.

3. A kit for detecting and quantifying a selected peptide or protein of interest (POI) (3) present in a biological sample **characterized by** comprising a biosensing device as described in any of the claims 1 to 2, a container with a peptide staining solution and preferably another container with a washing solution.

4. The kit according to claim 3 **characterized by** the staining solution comprises Coomassie brilliant blue R-250 (CB), bromophenol, methylene blue, silver nitrate and zinc nitrate, fluorescent dyes as eosin, rhodamine, and Nile blue oxazone.

5. The kit according to claim 3 **characterized by** the washing solution comprises acetate, citrate, phosphate or organic solvents as ethanol, methanol or in water.

6. A process for preparing a biosensing device as described in any of the claims 1 to 2 **characterized by**:
a) Providing a cellulose paper (CP) strip support,
b) Forming an amine layer (1) on the CP-strip support, by treating the CP-strip support with an amine compound selected from the group 3-aminopropyltriethoxysilane (APTES), ethylenediamine, phenylenediamine, poly(diallyldimethyl ammonium chloride, poly(vinylamine) hydrochloride, poly(L-lysine hydrobromide), polyethyleneimine hydrochloride, poly(allylamine hydrochloride), poly(4-aminostyrene), poly(allylamine), polyethylene glycol)bis(2-aminoethyl), chitosan, poly(I-lysine hydrobromide),
c) Incubating the amine layer (1) with a dialdehyde compound able to provide two terminal aldehyde functional groups (-CHO) forming an amine-aldehyde layer (2),
d) Binding a selected POI (3) to the amine-aldehyde layer of (c),
e) Molecularly imprinting of the POI (3) by polymerization of a monomeric structure (MON) around a template (4) with a cross-linker (CL) and an initiator (5),
f) Forming the imprinted sites (6) by removal of POI (3).

7. The process according to claim 6 **characterized by** the formation of the amine-layer (1) is performed by incubating the CP-strips in a solution 10% of the amine compound, wherein the amine compound is preferably 3-aminopropyltriethoxysilane (APTES).

8. The process according to claim 6 or 7 **characterized by** the formation of the aldehyde-layer (2) is performed by further incubation of the amine-layer (1) with a compound selected from glutaraldehyde (GLU), benzene-1,4-dialdehyde, and succindialdehyde, preferably glutaraldehyde (GLU).

9. The process according to any of the claims 6 to 8 **characterized by** the binding a selected POI (3) to the amine-aldehyde layer is performed by incubation of a solution of said POI (3) in an adequate buffer, such as PBS buffer at a pH value of approx. 7, for around 3h at a low temperature of 3 to 5°C, preferably of 3.5 to 4.5°C, more preferably at 4°C.

10. The process according to claim 6 **characterized by** the MIP is performed with a MON structure selected from acrylamide monomer (AAM), vinyl benzene boric acid, 4-vinyl benzaldehyde, 4-vinyl aniline, tert-butyl p-vinylphenylcarbonate, acrylic acid, methacrylic acid, trifluoromethyl acrylic acid methyl methacrylate, p-vinylbenzoic acid, itaconic acid, 4- ethylstyrene, styrene, 4-vinylpyridine, vinylpyridine, 1-vinylimidazole, acrylamide, 2-acrylamido-2-methyl-1-propane sulfonic acid, 44 2-hydroxyethyl methacrylate, trans-3-(3-pyridyl)-acrylic acid, the cross-linker (CL) is N,N'-methylenebis(acrylamide) (N,N-BISAA), bis-(1-(tert-butylperoxy)-1-methylethyl)-benzene, ethylene glycoldimethacrylate, N,N;-methylenediacrylamide, divinylbenzene;1,3-diisopropenyl benzene, N,N' -1,4-phenylenediacrylamine, 2,6-bisacryloylamidopyridine;3,5-bis(acryloylamido)benzoic acid, tetramethylene dimethacrylate, trimethylpropane trimethacrylate, and the initiator (5) is ammonium persulphate (APS), tetramethylethylenediamine(TEMED), azobisisobutyronitrile azobisdimethylvaleronitrile,4,40-azo(4-cyanovaleric acid), benzoylperoxide and dimethylacetal of benzyl.

11. A method for detecting and quantifying a POI in a sample **characterized by**:
a) Providing a biosensing device as described in any of the claims 1 to 2,
b) Contacting said biosensor with a biological sample to be tested for a given POI, for at least 30 minutes, preferably for 30 to 60 minutes,
c) Staining the biosensor with a peptide staining solution, and
d) Identifying a change of colour of the biosensing device, for the detection of a selected POI, and/or comparing the colour generated with a standard colour gradient for the corresponding peptide staining composition, for the quantification of a selected POI present in the sample to be tested.

12. The method according to claim 11 **characterized by** the sample to be tested is a sample of urine, blood, saliva, or sweat.

13. The method according to claim 11 **characterized by** the identification of the colour change of step (d) is made automatically by a computer program or application running in a processor unit by comparing the resulting colour change generated by the method as described in any of the claims 11 to 12 with a library of colours for different POIs at variable concentrations, and stained with different peptide-staining solutions.

## Patentansprüche

1. Ein Biosensing-Gerät zur Erkennung und zur Quantifizierung eines ausgewählten Peptids oder interessierenden Proteins (Protein of Interest, POI) (3), das in einer biologischen Probe vorhanden ist, **dadurch gekennzeichnet, dass** es eine Cellulosepapier(CP)-Teststreifenplattform umfasst, das eine Aminschicht (1) und eine biologische Erkennungsschicht für das genannte POI (3) umfasst, die durch die Technologie des molekularen Prägens von Polymeren (molecular imprinting polymer, MIP) hergestellt wird und in der Lage ist, nach Färbung mit einer geeigneten Peptid-Färbelösung eine Farbänderung zu erzeugen;
wobei die genannte Cellulosepapier-Teststreifenplattform ein qualitativer Filter vom Typ Papier ist;
wobei die genannte Aminschicht erhältlich ist durch Behandeln der Cellulosepapier(CP)-Teststreifenplattform mit einer Verbindung, die aus der folgenden Liste ausgewählt ist: 3-Aminopropyltriethoxysilan (APTES), Ethylendiamin, Phenylendiamin, Poly(Diallyldimethylammoniumchlorid, Poly(Vinylamin) Hydrochlorid, Poly(L-Lysin Hydrobromid), Polyethylenimin Hydrochlorid, Poly(Allylamin Hydrochlorid), Poly(4-Aminostyren), Poly(Allylamin), Polyethylenglykol)bis(2-aminoethyl), Chitosan, Poly(L-Lysin Hydrobromid);
wobei die genannte Aminschicht ferner mit einer Lösung inkubiert wird, die eine Dialdehydverbindung enthält, die in der Lage ist, zwei endständige funktionelle Aldehydgruppen bereitzustellen, wodurch eine Amin-Aldehyd-Schicht (2) gebildet wird,
wobei eine molekular geprägte Polymerschicht (MIP), die in der Lage ist, das genannte POI (3) zu erkennen, auf der genannten Amin-Aldehyd-Schicht aufgebaut ist; und
wobei das genannte POI (3) aus der folgenden Liste ausgewählt ist: Peptid Aß-42, kardiales Myoglobin, Troponin T, CK-MB Isoenzym, tumorassoziiertes MUC-1-Protein, Carzinoembryonales Antigen und Carbohydrat-Antigen (CA 19-9), neurodegenerative Biomarker wie Amyloid β42, Tau-Protein, Interleukin, Antikörper wie SARS-CoV-2 (2019-nCoV), Antikörper gegen das Malaria-, Zika- und Dengue-Virus und die entsprechenden Antigene, Virusproteine (SARS-CoV-2 (2019-nCoV) Antigen), Bakterienproteine wie Protein A und menschliches Serumalbumin.

2. Das Biosensing-Gerät nach dem vorangehenden Anspruch, **dadurch gekennzeichnet, dass** das Cellulosepapier (CP) aus einer Gruppe ausgewählt wird, umfassend Whatman^{®} Chromatographiepapier aus Cellulose, Whatman^{®} qualitatives Filterpapier, Grade 470, Filmstreifen aus Nitrocellulose, Whatman qualitative Filterpapier, rund, Grade 4, GE Healthcare Whatman^{™} Cellulose-Filterpapier und Cellulose- und Polyesterfaser-Reinraumtücher.

3. Ein Kit zur Erkennung und zur Quantifizierung eines ausgewählten Peptids oder interessierenden Proteins (POI) (3), das in einer biologischen Probe vorhanden ist, **dadurch gekennzeichnet, dass** es ein Biosensing-Gerät, wie in einem der Ansprüche 1 bis 2 beschrieben, einen Behälter mit einer Färbelösung für Peptide und bevorzugt einen weiteren Behälter mit einer Waschlösung umfasst.

4. Das Kit nach Anspruch 3, **dadurch gekennzeichnet, dass** die Färbelösung Coomassie-Brillant-Blau R-250 (CB), Bromphenol, Methylenblau, Silbernitrat und Zinknitrat sowie Fluoreszenzfarbstoffe wie Eosin, Rhodamin und Nilblau-Oxazon enthält.

5. Das Kit nach Anspruch 3, **dadurch gekennzeichnet, dass** die Waschlösung Acetat, Citrat, Phosphat oder organische Lösungsmittel wie Ethanol, Methanol oder Wasser enthält.

6. Ein Verfahren zur Vorbereitung eines Biosensing-Geräts, wie in einem der Ansprüche 1 bis 2 beschrieben, **gekennzeichnet durch**:
a) Bereitstellung eines Cellulosepapier(CP)-Streifenträgers,
b) Bildung einer Aminschicht (1) auf dem CP-Streifenträger durch Behandlung des CP-Streifenträgers mit einer Aminverbindung, ausgewählt aus der Gruppe 3-Aminopropyltriethoxysilan (APTES), Ethylendiamin, Phenylendiamin, Poly(Diallyldimethylammoniumchlorid, Poly(Vinylamin) Hydrochlorid, Poly(L-Lysin Hydrobromid), Polyethylenimin Hydrochlorid, Poly(Allylamin Hydrochlorid), Poly(4-Aminostyren), Poly(Allylamin), Polyethylenglykol)bis(2-aminoethyl), Chitosan, Poly(L-Lysin Hydrobromid),
c) Inkubation der Aminschicht (1) mit einer Dialdehydverbindung, die in der Lage ist, zwei endständige funktionelle Aldehydgruppen (-CHO) bereitzustellen, wodurch eine Amin-Aldehyd-Schicht (2) gebildet wird,
d) Bindung eines ausgewählten POI (3) an die Amin-Aldehyd-Schicht von (c),
e) Molekulare Prägung des POI (3) durch Polymerisation einer monomeren Struktur (MON) um eine Vorlage (4) mit einem Vernetzer (CL) und einem Initiator (5),
f) Bildung der geprägten Stellen (6) durch Entfernung des POI (3).

7. Das Verfahren nach Anspruch 6, **dadurch gekennzeichnet, dass** die Bildung der Aminschicht (1) durch Inkubation des CP-Streifens in einer 10%-igen Lösung der Aminverbindung erfolgt, wobei die Aminverbindung bevorzugt 3-Aminopropyltriethoxysilan (APTES) ist.

8. Das Verfahren nach Anspruch 6 oder 7, **dadurch gekennzeichnet, dass** die Bildung der Aldehydschicht (2) durch weitere Inkubation der Aminschicht (1) mit einer Verbindung, ausgewählt aus Glutaraldehyd (GLU), Benzol-1,4-dialdehyd und Succinaldehyd, bevorzugt Glutaraldehyd (GLU), erfolgt.

9. Das Verfahren nach einem der Ansprüche 6 bis 8, **dadurch gekennzeichnet, dass** die Bindung eines ausgewählten POI (3) an die Amin-Aldehyd-Schicht durch Inkubation einer Lösung des genannten POI (3) in einem geeigneten Puffer, wie z. B. PBS-Puffer mit einem pH-Wert von ca. 7, für etwa 3 Stunden bei einer niedrigen Temperatur von 3 bis 5°C, bevorzugt von 3,5 bis 4,5°C, besonders bevorzugt bei 4°C, erfolgt.

10. Das Verfahren nach Anspruch 6, **dadurch gekennzeichnet, dass** das MIP mit einer MON-Struktur durchgeführt wird, ausgewählt aus Acrylamidmonomer (AAM), Vinylbenzol Borsäure, 4-Vinyl Benzaldehyd, 4-Vinylanilin, tert-Butyl-p-vinylphenylcarbonat, Acrylsäure, Methacrylsäure, Trifluormethylacrylsäure Methylmethacrylat, p-Vinylbenzoesäure, Itaconsäure, 4-Ethylstyrol, Styrol, 4-Vinylpyridin, Vinylpyridin, 1-Vinylimidazol, Acrylamid, 2-Acrylamido-2-methyl-1-propansulfonsäure, 44 2-Hydroxyethylmethacrylat, trans-3-(3-Pyridyl)-acrylsäure, der Vernetzer (CL) N,N'-Methylenbis(acrylamid) (N,N-BISAA), Bis-(1-(tert-butylperoxy)-1-methylethyl)-benzol, Ethylenglycoldimethacrylat, N,N;-Methylendiacrylamid, Divinylbenzol, 1,3-Diisopropenylbenzol, N,N'-1,4-Phenylendiacrylamin, 2,6-Bisacryloylamidopyridin; 3,5-Bis(acryloylamido)benzoesäure, Tetramethylendimethacrylat, Trimethylpropantrimethacrylat, und der Initiator (5) Ammoniumpersulfat (APS), Tetramethylethylendiamin (TEMED), Azobisisobutyronitril, Azobisdimethylvaleronitril, 4,40-Azo(4-Cyanovaleriansäure), Benzoylperoxid und Dimethylacetal von Benzyl ist.

11. Ein Verfahren zur Erkennung und zur Quantifizierung eines POI in einer Probe, **gekennzeichnet durch**:
a) Bereitstellen eines Biosensing-Geräts nach einem der Ansprüche 1 bis 2,
b) Inkontaktbringen des genannten Biosensors mit einer biologischen Probe, die auf ein bestimmtes POI getestet werden soll, für mindestens 30 Minuten, bevorzugt für 30 bis 60 Minuten,
c) Färben des Biosensors mit einer Färbelösung für Peptide, und
d) Bestimmen einer Farbänderung des Biosensing-Geräts zur Erkennung eines ausgewählten POI und/oder Vergleichen der erzeugten Farbe mit einem Standardfarbgradienten für die entsprechende Färbezusammensetzung für Peptide zur Quantifizierung eines ausgewählten POI, das in der zu testenden Probe vorhanden ist.

12. Das Verfahren nach Anspruch 11, **dadurch gekennzeichnet, dass** die zu untersuchende Probe eine Urin-, Blut-, Speichel- oder Schweißprobe ist.

13. Das Verfahren nach Anspruch 11, **dadurch gekennzeichnet, dass** die Identifizierung der Farbänderung von Schritt (d) automatisch durch ein Computerprogramm oder eine Anwendung erfolgt, das/die in einer Prozessoreinheit abläuft, indem die sich ergebende Farbänderung, die durch das in einem der Ansprüche 11 bis 12 beschriebene Verfahren erzeugt wird, mit einer Farbbibliothek für verschiedene POls bei variablen Konzentrationen und gefärbt mit verschiedenen Färbelösungen für Peptide verglichen wird.

## Revendications

1. Un dispositif biosenseur pour détecter et quantifier un peptide ou une protéine d'intérêt (POI) (3) choisi(e) présent(e) dans un échantillon biologique **caractérisé en ce qu'**il comprend une plateforme de bandelette-test de papier de cellulose (CP) comprenant une couche d'amine (1), et une couche de bioreconnaissance pour ledit(ladite) POI (3) qui est créée à l'aide d'une technologie de polymères à empreinte moléculaire (PEM) et est capable de générer un changement de couleur après avoir été colorée avec une solution de coloration de peptide adéquate ;
dans lequel ladite plateforme de bandelette-test de papier de cellulose est un type de papier à filtre qualitatif;
dans lequel ladite couche d'amine peut être obtenue en traitant la plateforme de bandelette-test de papier de cellulose (CP) avec un composé choisi parmi une liste consistant en : 3-aminopropyltriéthoxysilane (APTES), éthylènediamine, phénylènediamine, poly(chlorure d'ammonium de diallyldiméthyle), poly(chlorhydrate de vinylamine), poly(bromhydrate de L-lysine), chlorhydrate de polyéthylènimine, poly(chlorhydrate d'allylamine), poly(4-aminostyrène), poly(allylamine), polyéthylèneglycol)bis(2-aminoéthyle), chitosan, poly(bromhydrate de I-lysine) ;
dans lequel ladite couche d'amine est également incubée avec une solution comprenant un composé dialdéhyde capable de fournir deux groupes fonctionnels aldéhydes terminaux, formant ainsi une couche d'amine-aldéhyde (2),
dans lequel une couche polymère à empreinte moléculaire (PEM) capable de reconnaitre ledit(ladite) POI (3) est construite au-dessus de ladite couche d'amine-aldéhyde ; et
dans lequel ledit(ladite) POI (3) est choisi(e) parmi une liste consistant en : peptide Aβ-42, myoglobine cardiaque, troponine T, isoenzyme CK-MB, protéine de cancer MUC-1, antigène carcinoembryonnaire et antigène glucidique (CA 19-9), biomarqueurs neurodégénératifs tels que l'amyloïde β42, protéine tau, interleukine, anticorps tels que le SARS-CoV-2 (2019-nCoV), anticorps contre la malaria, les virus zika et dengue et antigènes respectifs, protéines virales SARS-CoV-2 (antigène 2019-nCoV), protéines bactériennes telles que la protéine A, et sérum-albumine humaine.

2. Le dispositif biosenseur selon la revendication précédente **caractérisé en ce que** le matériau de papier de cellulose (CP) est choisi parmi un groupe comprenant des papiers de chromatographie en cellulose Whatman^{®}, du papier filtre qualitatif Whatman^{®}, Grade 470, des diapositives en film de nitrocellulose, des cercles de papier filtre qualitatif de Grade 4 Whatman, du papier filtre en cellulose GE healthcare Whatman^{™}, et des essuie-touts en papier pour salle blanche en fibre de polyester et cellulose.

3. Un kit pour détecter et quantifier un peptide ou une protéine d'intérêt (POI) (3) choisi(e) présent(e) dans un échantillon biologique **caractérisé en ce qu'**il comprend un dispositif biosenseur tel que décrit dans l'une quelconque des revendications 1 à 2, un récipient avec une solution de coloration de peptide et préférablement un autre récipient avec une solution de lavage.

4. Le kit selon la revendication 3 **caractérisé en ce que** la solution de coloration comprend du Bleu brillant de Coomassie R-250 (CB), du bromophénol, du bleu de méthylène, du nitrate d'argent et du nitrate de zinc, des colorants fluorescents tels que l'éosine, la rhodamine, et l'oxazone bleu de Nile.

5. Le kit selon la revendication 3 **caractérisé en ce que** la solution de lavage comprend de l'acétate, du citrate, du phosphate ou des solvants organiques tels que l'éthanol, le méthanol ou dans de l'eau.

6. Un procédé pour préparer un dispositif biosenseur tel que décrit dans l'une quelconque des revendications 1 à 2 **caractérisé par** les étapes suivantes :
a) fournir une bande de support en papier de cellulose (CP) ,
b) former une couche d'amine (1) sur la bande de support CP en traitant la bande de support CP avec un composé amine choisi parmi le groupe 3-aminopropyltriéthoxysilane (APTES), éthylènediamine, phénylènediamine, poly(chlorure d'ammonium de diallyldiméthyle), poly(chlorhydrate de vinylamine), poly(bromhydrate de L-lysine), chlorhydrate de polyéthylénimine, poly(chlorhydrate d'allylamine), poly(4-aminostyrène), poly(allylamine), polyéthylèneglycol)bis(2-aminoéthyle), chitosan, poly(bromhydrate de I-lysine) ,
c) incuber la couche d'amine (1) avec un composé dialdéhyde capable de fournir deux groupes fonctionnels aldéhydes terminaux (- CHO), formant une couche d'amine-aldéhyde (2),
d) lier un(e) POI (3) choisi(e) à la couche d'amine-aldéhyde de (c) ;
e) créer une empreinte moléculaire du(de la) POI (3) par polymérisation d'une structure monomère (MON) autour d'un modèle (4) avec un agent de réticulation (CL) et un initiateur (5) ;
f) former les sites imprimés (6) en retirant le(la) POI (3).

7. Le procédé selon la revendication 6 **caractérisé en ce que** la formation de la couche d'amine (1) est exécutée par incubation des bandes CP dans une solution de 10% du composé amine, dans lequel le composé amine est préférablement du 3-aminopropyltriéthoxysilane (APTES).

8. Le procédé selon la revendication 6 ou 7 **caractérisé en ce que** la formation de la couche aldéhyde (2) est exécutée par incubation additionnelle de la couche d'amine (1) avec un composé choisi parmi du glutaraldéhyde (GLU), du benzène-1,4-dialdéhyde, et du succindialdéhyde, préférablement du glutaraldéhyde (GLU).

9. Le procédé selon l'une quelconque des revendications 6 à 8 **caractérisé en ce que** la liaison d'un(d'une) POI (3) choisi(e) à la couche d'amine-aldéhyde est exécutée par incubation d'une solution dudit(de ladite) POI (3) dans un tampon adéquat, tel qu'un tampon PBS à valeur de pH d'environ 7, durant près de 3h à une température basse de 3 °C à 5 °C, préférablement de 3,5 °C à 4,5 °C, plus préférablement de 4 °C.

10. Le procédé selon la revendication 6 **caractérisé en ce que** le PEM est exécuté avec une structure MON choisie parmi un monomère d'acrylamide (AAM), acide borique de vinylbenzène, 4-benzaldéhyde de vinyle, 4-aniline de vinyle, tert-butyl p-vinylphénylcarbonate, acide acrylique, acide méthacrylique, acide acrylique trifluorométhyle méthacrylate de méthyle, acide p-vinylbenzoïque, acide itaconique, 4-éthylstyrène, styrène, 4-vinylpyridine, vinylpyridine, 1-vinylimidazole, acrylamide, acide 2-acrylamido-2-méthyl-1-propanesulfonique, 44 méthacrylate de 2-hydroxyéthyle, acide trans-3-(3-pyridyl)-acrylique, l'agent de réticulation (CL) est du N,N'-méthylenebis(acrylamide) (N,N-BISAA), bis-(1-(tert-butylperoxy)-1-méthyléthyl)-benzène, glycoldiméthacrylate d'éthylène, N,N'-méthylènediacrylamide, divinylbenzène ; benzène 1,3-diisopropényle, N,N'-1,4-phénylènediacrylamine, 2,6-bisacryloylamidopyridine ; acide 3,5-bis(acryloylamido)benzoïque, diméthacrylate de tétraméthylène, triméthacrylate de triméthylpropane, et l'initiateur (5) est du persulfate d'ammonium (APS), tétraméthyléthylènediamine (TEMED), azobisdiméthylvaleronitrile d'azobisisobutyronitrile, 4,40-azo(4-acide cyanovalérique), péroxyde de benzoyle et diméthylacétal benzylique.

11. Un procédé pour détecter et quantifier un(e) POI dans un échantillon **caractérisé par** :
a) fournir un dispositif biosenseur tel que décrit dans l'une quelconque des revendications 1 à 2 ;
b) mettre ledit biosenseur en contact avec un échantillon biologique pour qu'il soit testé pour un(e) POI donné(e), durant au moins 30 minutes, préférablement durant 30 à 60 minutes ;
c) colorer le biosenseur avec une solution de coloration de peptide, et
d) identifier un changement de couleur du dispositif biosenseur, pour la détection d'un(e) POI choisi(e), et/ou comparer la couleur générée à un dégradé de couleurs standard pour la composition de coloration de peptide correspondante, pour la quantification d'un(e) POI choisi(e) présent(e) dans l'échantillon à tester.

12. Le procédé selon la revendication 11 **caractérisé en ce que** l'échantillon à tester est de l'urine, du sang, de la salive, ou de la sueur.

13. Le procédé selon la revendication 11 **caractérisé en ce que** l'identification du changement de couleur de l'étape (d) est exécutée automatiquement par un logiciel informatique ou une application exécutée sur un processeur en comparant le changement de couleur résultant généré par le procédé tel que décrit dans l'une quelconque des revendications 11 à 12 à une gamme de couleurs pour différentes POIs à des concentrations variables, et coloré avec différentes solutions de coloration de peptide.
